# EUROPEAN PATENT APPLICATION

(11) **EP 4 036 248 A1**
(43) Date of publication of application: **03.08.2022**
(21) Application number: 21154220.4
(22) Date of filing: 29.01.2021
(51) Int. Cl.: C12Q 1/6806

(54) **METHOD FOR LIBRARY PREPARATION IN NEXT GENERATION SEQUENCING BY ENZYMATIC DNA FRAGMENTATION**

(71) Applicant: Miltenyi Biotec B.V. & Co. KG, 51429 Bergisch Gladbach (DE)
(72) Inventor: WAHL, Matthias, 51429 Bergisch Gladbach (DE)
(74) Representative: Kisters, Michael Marcus

(57) **Abstract**

The invention is directed to a method for obtaining a nucleic acid library of a sample comprising polynucleotides comprising the steps
a. multiplying the polynucleotides by a polymerase
b. fragmenting the multiplied polynucleotides by creating nicks
c. coupling an oligonucleotide sequence to the nicks to create the target library
**characterized in that**
step a) is performed by providing A, T, G, C and U nucleotides wherein the molar ratio of T and U is between 150:1 and 25:1 and step b) is performed by excision of the U nucleotides.

## Description

### BACKGROUND

Next Generation Sequencing is an emerging technology extending to all areas of Biomedical Research and Clinical Diagnostics. One of the key steps in Next Generation Sequencing is the Library Preparation (Library Prep).

During Library Prep, the DNA to be sequenced is provided with specific sequences on both ends (adaptor sequences), to which the sequencing primer or amplification primers bind. To these adaptor sequences further sequences providing other information may be added, like specific sequences (barcodes) for the assignment of a Next Generation Sequencing read to a particular sample or a cell or a molecule.

Many Next Generation Sequencing assays require that the DNA of interest is being fragmented. Fragmentation techniques are for example disclosed in:

Hess JF, Kohl TA, Kotrová M, Rönsch K, Paprotka T, Mohr V, Hutzenlaub T, Brüggemann M, Zengerle R, Niemann S, Paust N. Library preparation for next generation sequencing: A review of automation strategies. Biotechnol Adv. 2020 Jul-Aug;41:107537. doi: 10.1016/j.biotechadv.2020.107537. Epub 2020 Mar 19. PMID: 32199980.

Head SR, Komori HK, LaMere SA, et al. Library construction for next-generation sequencing: overviews and challenges. Biotechniques. 2014;56(2):61-passim. Published 2014 Feb 1. doi:10.2144/000114133

The known fragmentation techniques include:
- Physical shearing of nucleic acids, for example using the Covaris ultra-sonicator.
- Enzymatical fragmentation of nucleic acids using nucleases.
- Fragmentation of nucleic acids with the use of transposase, which semi-randomly inserts adaptor sequences into DNA.

Physical shearing requires the purchase of an expensive instrument, cannot be automated, and cannot be parallelized for multiple samples when using a single instrument.

The enzymatic fragmentation and tagmentation procedures have a significant disadvantage: The degree of fragmentation and tagmentation is very sensitive towards time and the input amount (input DNA), therefore this step has to be very tightly controlled (by accurate quantification of input amount and incubation time), and reagents have to be prechilled in order to avoid that the reaction starts prematurely.

The requirement that reactions have to be pipetted on ice provides a significant usability constraint, since many laboratories are not equipped with equipment for chilling reagents (especially diagnostics labs using multiple physically separated rooms for contamination prevention refrain from using equipment for chilling reagents like ice machines). Also, incubations at low temperature make automation of the pipetting steps challenging because not every automation solution is capable of cooling reagents.

The time criticality of incubation steps also impacts the scalability of workflows as some of the reactions will start immediately after addition of the sample.

The methods of the prior art are compared in the following table

| | Shearing | Tagmentation | Enzymatic fragmentation using nucleases |
|---|---|---|---|
| Sequence bias | Low | Sequence bias: strong bias in low GC regions for Illumina Nexteraq kits using mutated Tn5 transposase | Lower sequence bias compared to Tagmentation |
| Importance of accurate quantification | | High | High |
| Importance of controlled time and temperature | | High | High |
| Multistep single- tube workflows possible? | Yes: End repair and Ligation can be conducted in single tubes | Yes | Very difficult: majority of workflows require multiple cleanup steps |
| Automated workflows possible? | No - shearing has to be conducted manually | Difficult: requires accurate quantification of low conc. DNA; requires time- and temperature controlled pipetting | Difficult: requires accurate quantification of low conc. DNA; requires time- and temperature controlled pipetting |

In order to avoid the downsides of the known methods, it is proposed to use dUTP (deoxyuridine triphosphate) and enzymes catalyzing the excision of uracil nucleotides for fragmenting DNA during the Library Prep workflow of a Next Generation Sequencing assays.

### SUMMARY

It was therefore an object of the invention to provide a method for obtaining a nucleic acid library of a sample comprising polynucleotides comprising the steps
a. multiplying the polynucleotides by a polymerase
b. fragmenting the multiplied polynucleotides by creating nicks
c. coupling an oligonucleotide sequence to the nicks to create the target library
**characterized in that**
step a) is performed by providing A, T, G, C and U nucleotides wherein the molar ratio of T and U is between 150:1 and 25:1 and step b) is performed by excision of the U nucleotides.

As A, T, G, C and U nucleotides, the known building blocks for oligonucleotide synthesis like dUTP nucleotides can be used.

Preferable, the A, T, G, C and U nucleotides are provided as Adenosine 5'-Triphosphate (ATP), 2'-Deoxyadenosine 5'-Triphosphate (dATP), Thymidine 5'-Triphosphate (TTP), 2'-Deoxythymidine 5'-Triphosphate (dTTP), Guanosine 5'-Triphosphate (GTP), 2'-Deoxyguanosine 5'-Triphosphate (dGTP), Cytidine 5'-Triphosphate (GTP) and 2'-Deoxycytidine 5'-Triphosphate (dGTP), 2'-Deoxyuridine, 5'-Triphosphate (dUTP) or Uridine-5'-triphosphate (UTP). The person skilled in the art is aware that these compounds are available as natural occurring form or chemicaly modified as derivative. In the method of the invention, the natural occurring form of the nucleotides and/or a derivative thereof (i.e. chemicaly modified version can be used.

The target nucleic acid library obtained by method of the invention may be sequenced. The method for sequencing is not particular important and any method for sequencing known in the art can be used for this purpose.

The oligonucleotide sequence coupled to the nicks is preferable an adaptor or primer sequence like a PCR starter sequence which can be used for amplification purposes, or a sequencing primer binding sequence which can be used for sequencing the target nucleic acid library.

### BRIEF DESCRIPTION OF THE DRAWINGS

Fig. 1 shows the principle of the method of the invention in a generic process.
Fig. 2 depicts a variant using messenger RNA as starting material
Fig. 3 depicts a variant using targeted enrichment (specific amplification) of one or multiple nucleic acid targets
Fig. 4 depicts a variant using linear amplification for the amplification of nucleic acids in the presence of dUTP nucleotides with Phi29 polymerase
Fig. 5 depicts a method for generating a nucleic acid library using nucleic acids fragmented with the method of invention by generating blunt ends followed by ligation of a specific nucleotide adapter.
Fig 6 depicts multiple different adaptor designs that can be used for the method shown in Figure 5.
Fig. 7 shows a variant of the method wherein the nucleic acid fragments are first denatured to obtain single-stranded nucleic acid fragments which are then provided with a specific nucleotide adapter .
Fig. 8 shows a variant of the method wherein the nucleic acid fragments are first denatured to obtain single-stranded nucleic acid fragments which are then ligated with poly-A tails at the 3' ends
Fig. 9 to 11 show experimental results

### DETAILED DESCRIPTION

The method of the invention provides a novel approach for statistical fragmenting of polynucleotides that can be utilized for the generation of sequencing libraries derived from target nucleic acids.

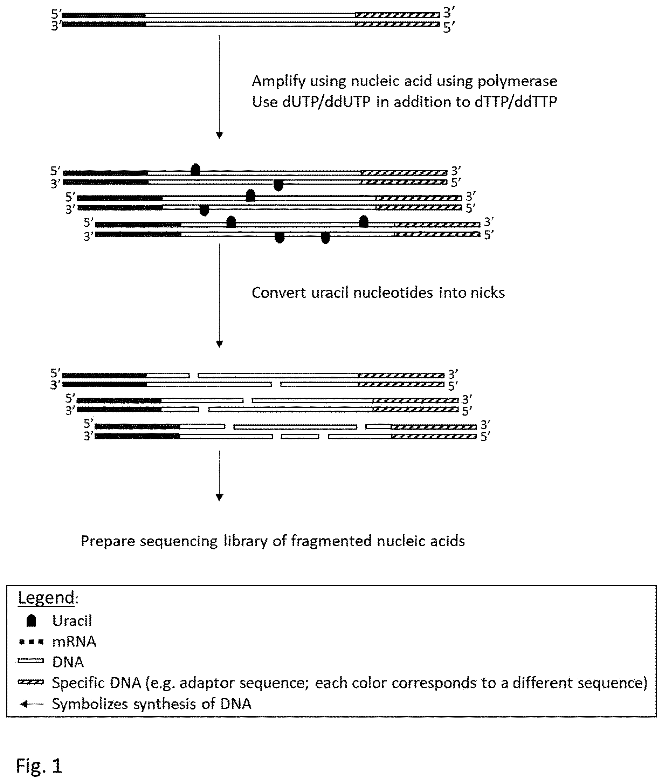

Instead of methods of prior art which use physical shearing, nucleases or transposases for statistical fragmentation of target nucleic acids, this method incorporates uracil nucleotides during a polymerisation step which subsequently are converted into nicks (Fig. 1).

A key step of the method is the initial polymerisation step which is already part of many nucleic acid library preparation methods. During this polymerisation step, dUTP or ddUTP nucleotides are incorporated into the polynucleotides being synthesized.

In the method of the invention, the target nucleic acids may be derived from genomic DNA, RNA or a plurality of DNA molecules comprising 50 to 2000 nucleotides.

### Step a - multiplying the target nucleic acids

Preferable before step a, the target nucleic acids are provided at the 3' and 5' ends with primer sequences for amplification.

Multiple polymerase-based methods exist which can be used for incorporating uracil bases into nucleic acid. The following sections contain three different methods that already are part of many workflows for the generation of sequencing libraries:
- Incorporation of uracil nucleotides during cDNA synthesis
- Incorporation of uracil nucleotides during PCR amplification
- Incorporation of uracil nucleotides during linear amplification

### Incorporation of uracil bases during cDNA synthesis:

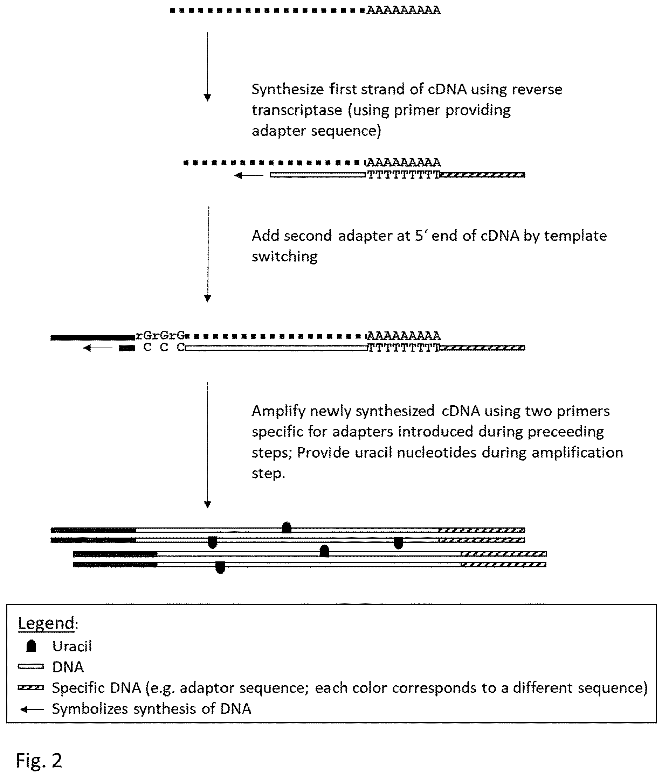

Fig. 2 depicts a method using messenger RNA as starting material. First, cDNA is synthesized using reverse transcriptase and an oligo(dT) primer (oligonucleotide with multiple T nucleotides); the oligo(dT) primer may contain one or more additional nucleotides at the 3' end; the oligo(dT) primer may also contains a specific nucleic acid sequence 5' to the oligo(dT) stretch (adaptor 1 containing a specific primer binding sequence 1; this adaptor is depicted with upward diagonal stripes).

Once the reverse transcriptase reaches the 5' end of the mRNA, a second specific sequence is introduced (adaptor 2 containing specific primer binding sequence 2; this adaptor is depicted with a solid box) using the template switching approach (Chenchik et al., 1998).

The two specific primers may also be introduced using random priming during reverse transcription and/ or during a subsequent second strand cDNA synthesis step.

This newly synthesized cDNA is then amplified in the presence of dUTP by a polymerase using primers specific to the primers incorporated during the cDNA synthesis. Alternatively, UTP or dUTP nucleotides may already be added during the reverse transcription and/ or second strand synthesis step; in this case, the amplification step may be omitted.

### Incorporation of uracil bases during PCR amplification:

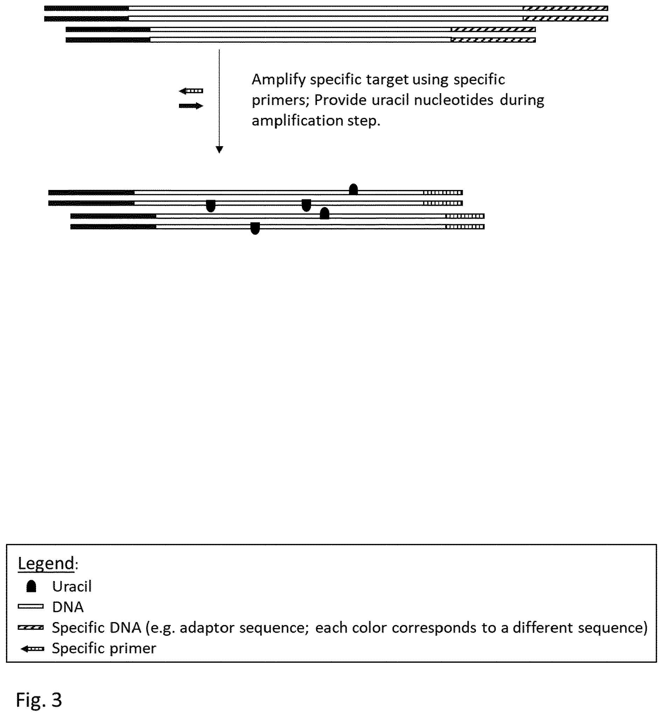

Preferable, step a) is conducted by polymerase chain reaction. Fig. 3 depicts an method using targeted enrichment (specific amplification) of one or multiple nucleic acid targets; in this example, the target enrichment is conducted by using one primer specific to a sequence already present in the template nucleic acid, and a second primer specific to the target or targets of interest. The targeted amplification is conducted using specific primers, a polymerase and nucleotides, including dUTPs.

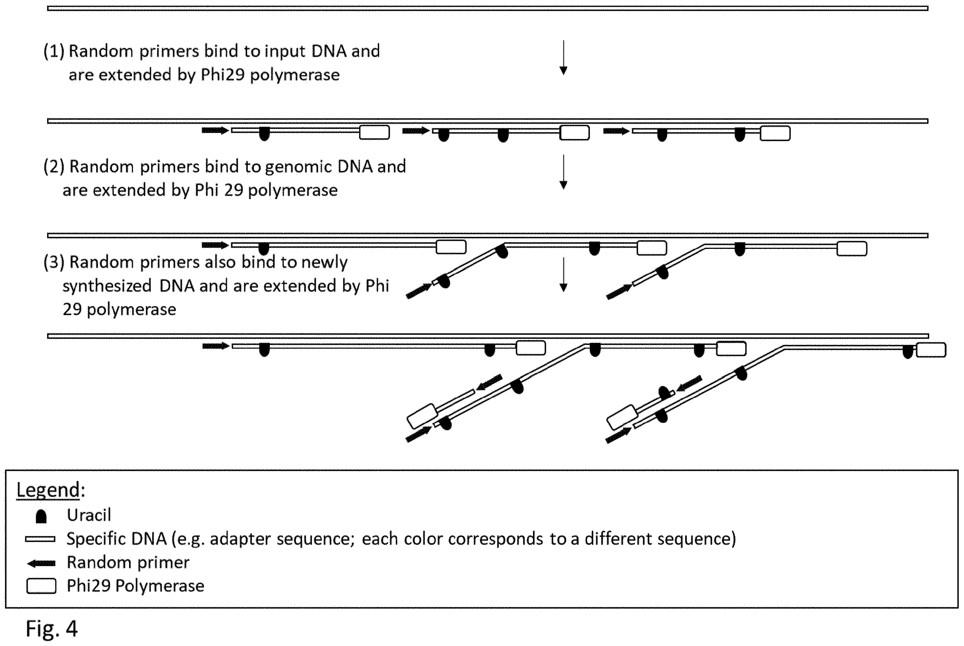

The amplification steps mentioned in the descriptions for Fig 3 and Fig 4 can be conducted by polymerase chain reaction using *Taq* polymerase (thermostable DNA polymerase I of *Thermus aquaticus*) or other proof-reading polymerases capable of mediating polymerase chain reactions. Alternatively, the amplification can be achieved using Loopmediated isothermal amplification.

### Incorporation of uracil bases during linear amplification:

Fig. 4 depicts a method using linear amplification for the amplification of nucleic acids in the presence of dUTP nucleotides, for example using Phi29 polymerase for the amplification of whole genomes (Silander et al., 2008).

### Step b - fragmentation of the polynucleotides

The newly synthesized nucleic acids are subsequently treated with an enzyme mixture capable of removing uracil nucleotides thereby creating nicks.

Preferable, nicks are generated by a providing one or more enzymes selected from the group consisting of DNA glycosylases (for example Uracil DNA Glycosylase), endonucleases (for example Endonuclease III or Endonuclease VIII), or engineered recombinant proteins (for example USER enzyme and thermolabile USER II enzyme).

Examples for such enzyme mixtures are uracil-DNA glycosylase (UDG) and endonuclease III or UDG and endonuclease VIII (Melamade et al, 1994; Jiang et al, 1007). Alternatively, commercial enzymes or enzyme mixes like the USER enzyme or the thermoliable USER enzyme from New England Biolabs may be used (Cat. No M5508 and M5507, New England Biolabs, Ipswich, MA, USA).

In attrition to providing enzymes, the creation of nick can be performed be applying elevated temperatures of chemicals.

The number of uracil bases in the newly synthesized nucleic acids can be tuned by adjusting the ratio between dUTP/ ddUTP and dTTP/ ddTTP nucleotides during the polymerisation step. The higher the relative abundance of dUTP/ ddUTP, the more uracil nucleotides will be incorporated (replacing thymidine nucleotides).

Since nicks are specifically generated at the sites of uracil nucleotides, the fragment length is proportional to the relative abundance of dUTP/ ddUTP during the polymerization step. Therefore, the fragment length can be statistically tuned by adjusting the relative abundance of dUTP/ ddUTP in the polymerization step.

### Step c - coupling oligonucleotides to the nicks

This section lists multiple preferred embodiments for creating nucleic acid libraries from nucleic acid fragments generated by incorporation of uracil nucleotides and subsequent excision of these uracil nucleotides.

Optionally, the oligonucleotide sequences coupled to the nicks are primer sequences.

To exemplify these embodiments, nucleic acid fragments generated using the method introduced in Fig. 2 (mRNA converted to cDNA with 5' and 3' specific adaptors introduced using template switching and oligo(dT) priming, respectively) are depicted.

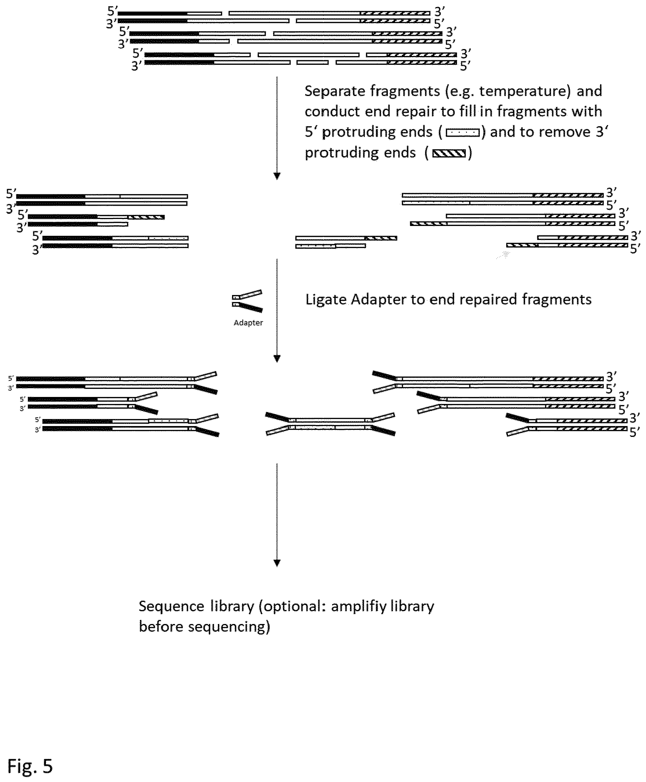

The embodiment shown in Fig. 5 first creates blunt ends to which a specific oligonucleotide adaptor is subsequently ligated. This is achieved by separating the fragmented nucleic acids followed by the treatment of the fragmented nucleic acids with an enzyme or an enzyme mix exhibiting a 5' → 3' polymerase activity and a 3' → 5' exonuclease activity.

At fragments with 5' protruding ends, a reverse complimentary second strand is being synthesized using the 5' → 3' polymerase activity ("fill-in"); at fragments with 3' protruding ends, the protrusion is removed using the 3' → 5' exonuclease activity. After this treatment, all fragments have blunt ends.

In a modification of this embodiment, one or more A nucleotides are added to the 3' end of the fragments ("A-tailing"). This A-tailing is achieved by either using an enzyme with A-tailing activity for the reaction above, or by an additional treatment with an enzyme exhibiting A-tailing activity.

Next, a double-stranded oligonucleotide (adaptor) is ligated to the fragments (either through blunt end ligation or with a double stranded oligonucleotide containing a T overhang in case the fragments were treated with an enzyme with A-tailing activity). The double-stranded adaptor used for ligation contains one or two specific primer binding sequences. In a modification of this embodiment, the adapter might be partially single-stranded.

In a variant of the invention, the nucleic acid library may be sequenced. For this purpose, the primer sequence/ these primer sequences added during adapter ligation can be used for subsequent sequencing of the nucleic acid library.

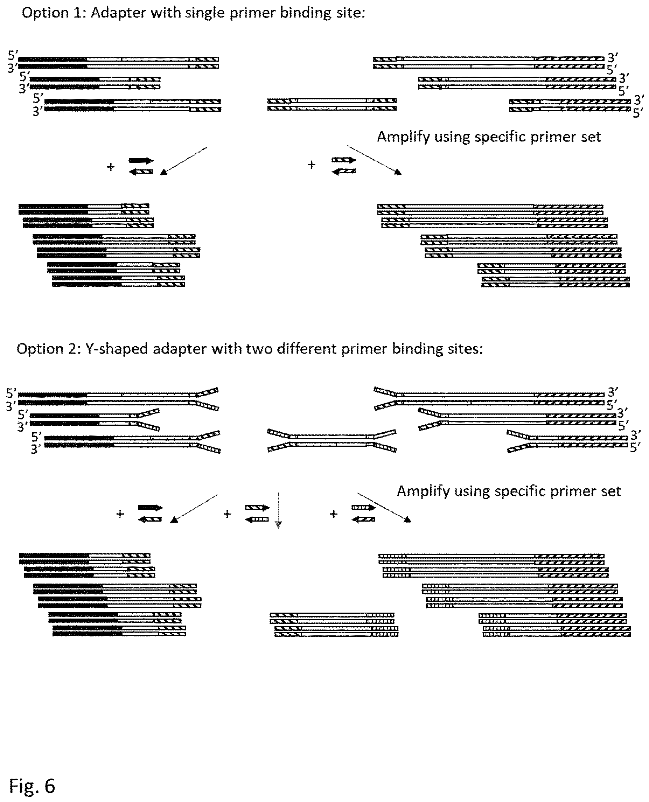

Optionally, the sequence library can be amplified before sequencing. Through the design of the adaptor, specific parts of the nucleic acid fragments can be amplified. Fig 6 depicts multiple different adaptor designs.

In one embodiment (option 1), an adaptor with a single primer binding sequence (specific primer binding site 3; depicted with downward diagonal stripes) is ligated to the nucleic acid fragments.

After ligation, the library fragments containing the 5' end of the original fragment can be specifically amplified using primers specific to primer binding sequence 2 and 3.

Library fragments containing the 3' end of the original fragment can be specifically amplified using primers specific to primer binding sequence 1 and 3.

The intermediate fragments will not efficiently amplify, as fragments with the same primer binding sequences (primer binding sequence 3) will form intramolecular hairpins, which prevent the binding of primers to the primer binding sits.

In another embodiment (option 2), a Y-shaped adaptor with two different primer binding sequences (specific primer binding site 3; depicted with downward diagonal stripes, and specific primer binding site 4; depicted with vertical stripes) is ligated to the nucleic acid fragments.

After ligation, the library fragments containing the 5' end of the original fragment can be specifically amplified using primers specific to primer binding sequence 2 and 3.

Library fragments containing the 3' end of the original fragment can be specifically amplified using primers specific to primer binding sequence 1 and 4.

The intermediate fragments can be amplified using primers specific to primer binding sequences 3 and 4.

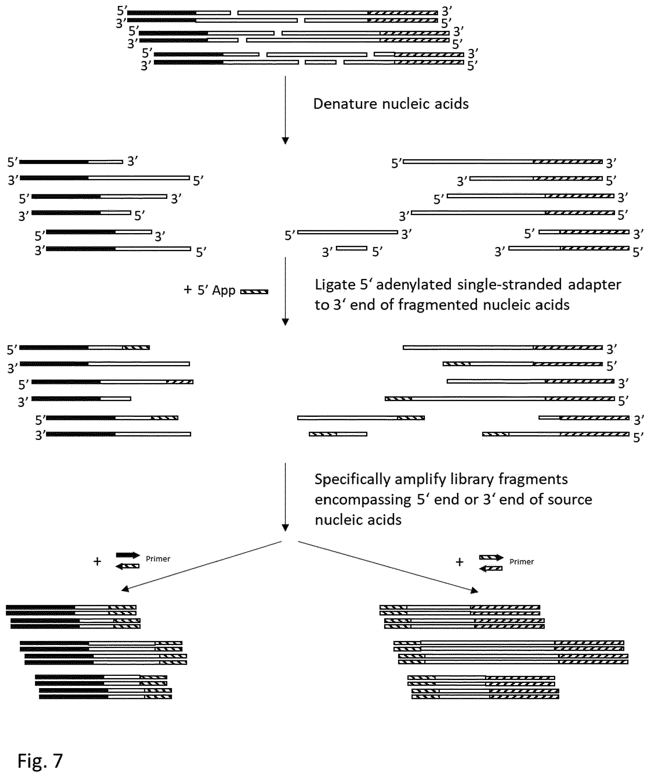

In another preferred embodiment depicted in Fig. 7, the nucleic acid fragments are first denatured (e.g. using heat or by increasing the pH): thereby, the nucleic acid fragments become single-stranded.

Next, a single-stranded oligonucleotide containing a specific primer binding site (adaptor 3 with primer sequence 3, depicted with downward diagonal stripes) is ligated to the 5' end of the single stranded nucleic acid fragments.

In the embodiment depicted in Fig. 7, the oligonucleotide has a 5' adenylation modification at the 5' end (5' App). The ligation reaction is catalysed using the Thermostable 5' App DNA/RNA Ligase from New England Biolabs (Cat. No M0319, New England Biolabs, Ipswich, MA, USA) or an equivalent enzyme.

The resulting nucleic acid library can either be sequenced directly or amplified using specific primer sets.

By the choice of the amplification primers, it is possible to amplify a subset of the nucleic acid library: primer sequence 1 (depicted with upward diagonal stripes) and primer sequence 3 (downward diagonal stripes) for the amplification of the fragments containing the 3' end of the original cDNA, and primer sequence 2 (solid) and primer sequence 3 for the amplification of fragments containing the 5' end, respectively.

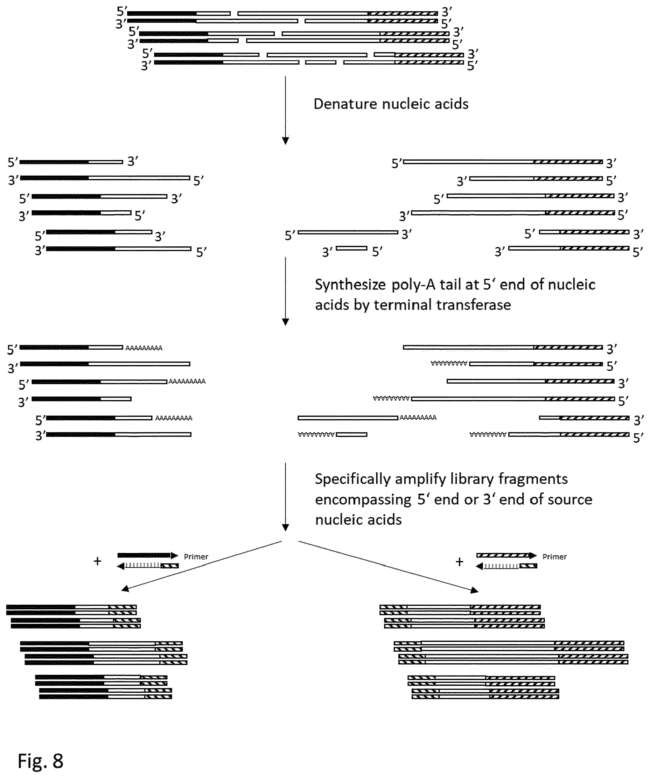

In a third preferred embodiment depicted in Fig. 8, the nucleic acid fragments are first denatured (e.g. using heat or by increasing the pH): thereby, the nucleic acid fragments become single-stranded.

Next, the single-stranded nucleic acid fragments are incubated with terminal transferase and a single oligonucleotide, thereby creating a mononucleotide tail at the 3' end of the nucleic acid fragments.

In the embodiment shown in Fig. 8, the nucleotide is ATP, resulting in a poly-A tail at the 3' end of the nucleic acid fragments.

In the next step, the fragments containing the 5' end of the original fragment can be amplified by a specific primer with a poly-T stretch at the 3' end of the primer (which binds to the poly-A tail of the library) and a primer specific for sequence 2 [depicted in solid]; the fragments containing the 3' end of the original fragment can be amplified using the same poly-T stretch containing primer and a primer specific for sequence 1 [upward diagonal stripes]).

### References

Melamede RJ, Hatahet Z, Kow YW, Ide H, Wallace SS. Isolation and characterization of endonuclease VIII from Escherichia coli. Biochemistry. 1994 Feb 8;33(5):1255-64. doi: 10.1021/bi00171a028. PMID: 8110759.

Jiang D, Hatahet Z, Melamede RJ, Kow YW, Wallace SS. Characterization of Escherichia coli endonuclease VIII. J Biol Chem. 1997 Dec 19;272(51):32230-9. doi: 10.1074/jbc.272.51.32230. PMID: 9405426.

Chenchik A., Zhu,Y.Y., Diatchenko,L., Li,R., Hill,J. and Siebert,P.D. (1998) Generation and use of high-quality cDNA form small amounts of total RNA by SMART PCR. In Siebert,P. and Larrick,J. (eds), Gene Cloning and Analysis by RT-PCR. Biotechniques Books, Natick, MA, pp. 305-319.

Silander K, Saarela J. Whole genome amplification with Phi29 DNA polymerase to enable genetic or genomic analysis of samples of low DNA yield. Methods Mol Biol. 2008;439:1-18. doi: 10.1007/978-1-59745-188-8_1. PMID: 18370092.

### EXAMPLES

### Example 1: The fragment size can be adjusted by the ratio between dUTP and dTTP during amplification

We first assessed whether the fragment size can be adjusted by the ratio between dUTP and dTTP in a polymerase chain reaction. As model system we chose amplified cDNA generated with the template switching approach shown in Figure 2 (generated using the Chromium Next GEM Single Cell V(D)J Reagent Kits v1.1, 10x Genomics, Pleasanton, CA, USA).

In order to statistically incorporate dUTP nucleotides, we re-amplified the cDNA for 10 cycles using the Q5U Hot Start High-Fidelity DNA Polymerase (Cat. No. M0493, New England Biolabs, Ipswich, MA, USA) according to the manufacturer's protocol. Four different relative amounts of dUTP were added to the reaction together with a no dUTP control (the percentage of dUTP refers to the fraction of dTTP replaced by dUTP in the reaction setup): condition 1: 20% dUTP, 80% dTTP; condition 2: 4% dUTP, 96% dTTP; condition 3: 0.8% dUTP, 99.2% dTTP; Condition 4: 0.16% dUTP, 99.84% dTTP; Condition 5: dTTP only.

After the amplification, an aliquot of the samples was treated with the Thermoliable USER II Enzyme (Cat. No. M5508, New England Biolabs, Ipswich, MA, USA) at 37°C for 15 minutes followed ("USER treatment") by a heat inactivation step at 65°C for 10 minutes. Samples were purified using 0,8x SPRIselect beads (Cat. No. B23317, Beckman Coulter, Brea, CA, USA) and analyzed on an Agilent 4200 TapeStation System using D5000 or High Sensitivity D5000 Screen Tapes (Cat. No. 5067-5588 and 5067-5592, Agilent, Santa Clara, CA, USA).

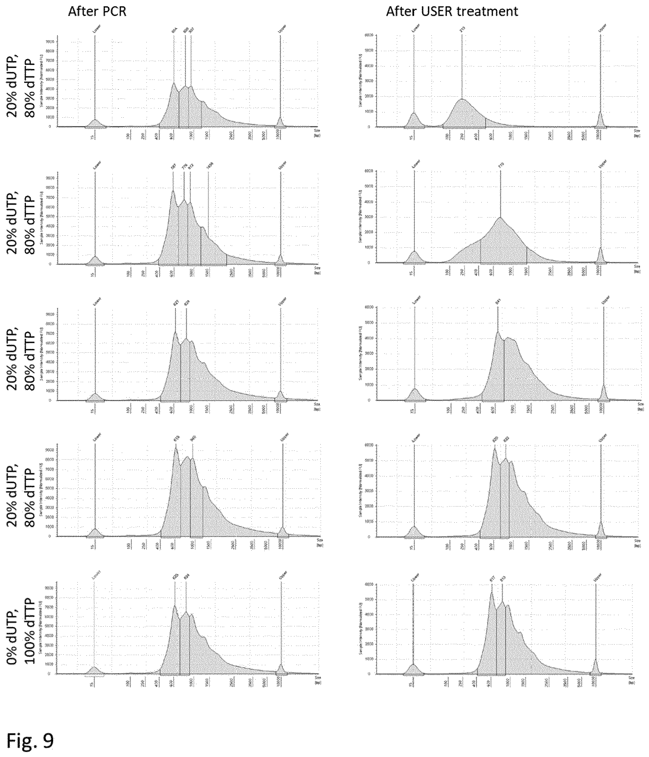

As seen in Figure 9, the presence of dUTP at different relative fractions did not impact the size distribution of the sample after amplification (left column); after USER treatment, the amplification products were fragmented, and the fragment size was inversely proportional to the relative fraction of dUTP (right column): the larger the relative fraction of dUTP, the smaller the statistical fragment size.

### Example 2: The fragment size is independent of the template input amount.

We next assessed whether the fragment size after USER treatment is dependent on the number of molecules used as input for the amplification reaction.

Two different input amounts were used for the initial amplification (0.5x template: 1 pg/µl; and 2x template: 4 pg/µl). Amplification and USER II treatment were conducted as described in example 1 with the exception that different relative amounts of dUTP were used: condition 1: 20% dUTP, 80% dTTP; condition 2: 10% dUTP, 90% dTTP; condition 3: 5% dUTP, 95% dTTP; condition 4: 2.5% dUTP, 97.5% dTTP.

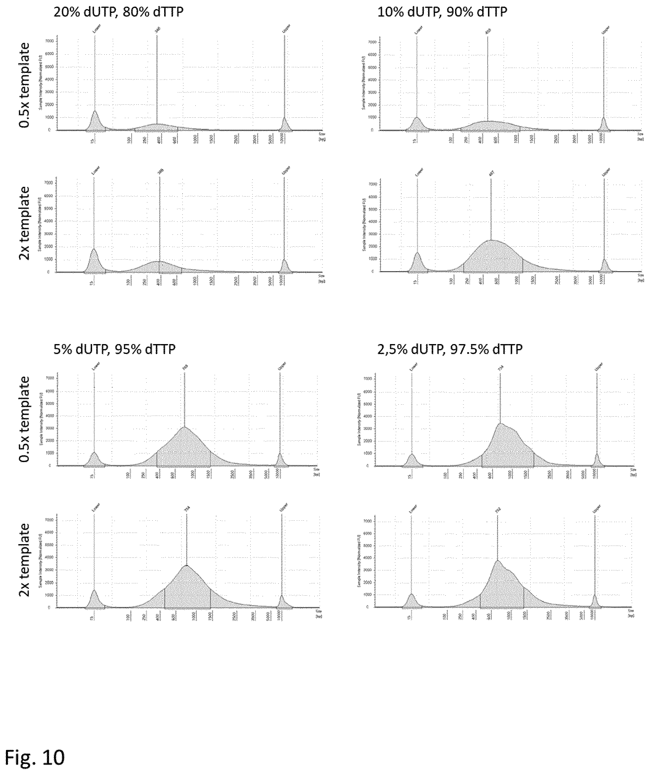

Figure 10 shows the results for the two different template concentrations after USER treatment: for all dUTP concentrations, the fragment distribution was very similar independent of the template concentration. This proves that the proposed method has the very unique feature that the statistical size of nucleic acid fragment does not depend on the input amount. Instead, the fragment size can be fine-tuned by adjusting the relative abundance of dUTP in an amplification reaction. This is a very unique property which facilitates workflows that do not depend on accurate quantification of the starting material or intermediate products.

### Example 3: Next Generation Sequencing libraries generated with dUTP/ USER fragmented nucleic acids

In this example we amplified re-amplified the same template used in example 1 and 2 in 25 µl reactions (template concentration: 1 pg/µl). Amplification and USER treatment were conducted as described in example 1, and the relative amounts of dUTP were identical to experiment 2: condition 1: 20% dUTP, 80% dTTP; condition 2: 10% dUTP, 90% dTTP; condition 3: 5% dUTP, 95% dTTP; Condition 4: 2.5% dUTP, 97.5% dTTP.

10 out of the 25 + 1 µl were subjected to a end repair and A-tailing reaction by using the NEBNext^{®} Ultra^{™} II End Repair/dA-Tailing Module (Cat. No. E7546, New England Biolabs, Ipswich, MA, USA) following the manufacturers instruction (at half scale), followed by the ligation of the 10x genomics Adaptor Mix (PN 220026, 10x Genomics, Pleasanton, CA, USA) using the NEBNext^{®} Ultra^{™} II Ligation Module (Cat. No. E7595, New England Biolabs, Ipswich, MA, USA; also at half scale).

The remaining 16 µl of the amplification/ Thermoliable USER II reaction were purified using 1x SPRIselect beads (Cat. No. B23317, Beckman Coulter, Brea, CA, USA). The samples were eluted in 25 µl elution buffer and subjected to the same procedure of end repair and A-tailing, and adaptor ligation as described for the non-purified counterpart.

Two µl of each pair of samples were subjected to 10 cycles of sample index PCR using the reagents taken from the Chromium Single Cell 5' Library Construction Kit (PN-1000020, 10x Genomics, Pleasanton, CA, USA). Additionally, 10µl of the ligation product derived from the sample already purified after the amplification/ USER II step was also purified using 1x SPRIselect beads (Cat. No. B23317, Beckman Coulter, Brea, CA, USA) and subjected to the same sample index PCR protocol.

All samples were finally purified using 0.8x SPRIselect beads (Cat. No. B23317, Beckman Coulter, Brea, CA, USA).

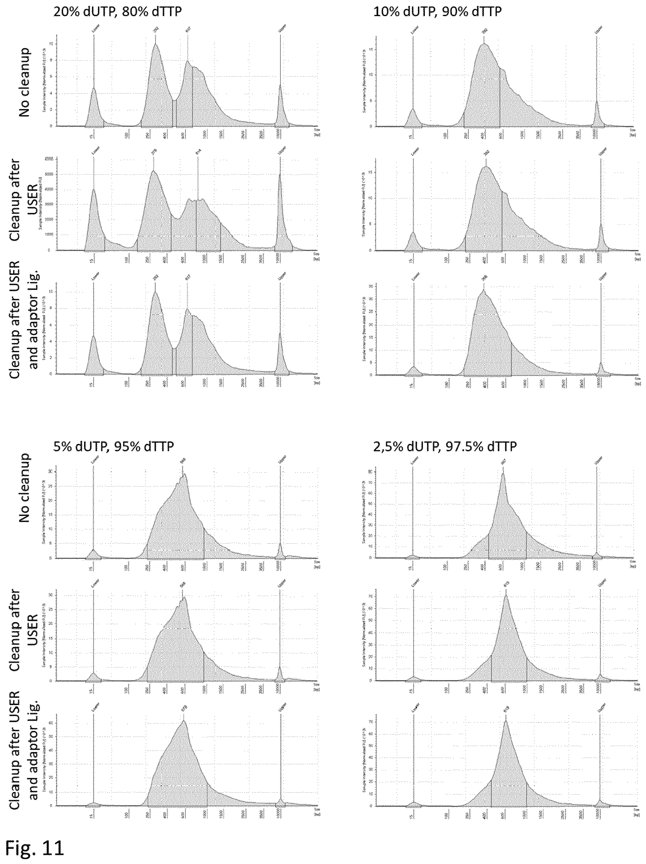

The result summarized in Figure 11 proves that libraries can be generated with nucleic acid fragments generated with the proposed method, and that the library size is inversely proportional to the relative fraction of dUTP in the initial PCR reaction.

### It additionally shows that all three procedures:

No purification after amplification/ USER II treatment, no purification after ligation

Purification after amplification/ USER II treatment, no purification after ligation

Purification after amplification/ USER II treatment, purification after ligation gave rise to libraries of similar size distribution.

This observation is of great importance, as it exemplifies an additional unexpected advantage of the proposed method: During the different workflow steps, little undesired artifacts are being generated that compete with the amplification of the final library (sample index PCR), therefore the majority of amplicons generated is specific. Because of this, only one single purification (cleanup) is required to deplete fragments that are too small.

In contrast, methods of the art like the Chromium Single Cell 5' Library Construction Kit (PN-1000020, 10x Genomics, Pleasanton, CA, USA) require a total of three cleanup steps and one size selection step, which are time consuming and lead to challenges when automating an next generation sequencing workflow.

## Claims

1. A method for obtaining a nucleic acid library of a sample comprising polynucleotides comprising the steps
d. multiplying the polynucleotides by a polymerase
e. fragmenting the multiplied polynucleotides by creating nicks
f. coupling an oligonucleotide sequence to the nicks to create the target library
**characterized in that**
step a) is performed by providing A, T, G, C and U nucleotides wherein the molar ratio of T and U is between 150:1 and 25:1 and step b) is performed by excision of the U nucleotides.

2. Method according to claim 1 **characterized in that** the A, T, G, C and U nucleotides are provided as Adenosine 5'-Triphosphate (ATP), 2'-Deoxyadenosine 5'-Triphosphate (dATP), Thymidine 5'-Triphosphate (TTP), 2'-Deoxythymidine 5'-Triphosphate (dTTP), Guanosine 5'-Triphosphate (GTP), 2'-Deoxyguanosine 5'-Triphosphate (dGTP), Cytidine 5'-Triphosphate (GTP) and 2'-Deoxycytidine 5'-Triphosphate (dGTP), 2'-Deoxyuridine, 5'-Triphosphate (dUTP) or Uridine-5'-triphosphate (UTP) or a derivate thereof.

3. Method according to claim 1 or 2 **characterized in that** after step b), the nicks are provided with a polymerase exhibiting 5' → 3' exonuclease activity, thereby filling in the 3' recessing ends and removing the 5' overhangs of the nicks.

4. Method according to any of the claims 1 to 3 **characterized in that** step c) is performed by providing a ligase.

5. Method according to any of the claims 1 to 4 **characterized in that** after step b), the nicks are denaturated into single strand nicks and the single strand nicks are provided with a ligase which couples oligonucleotide sequences to the 3' end of the single strand nicks.

6. Method according to c any of the claims 1 to 4 **characterized in that** after step b), the nicks are denaturated into single strand nicks and the single strand nicks are provided with a terminal transferase which couples homonucleotides comprising 2 to 20 nucleic acids as oligonucleotide sequences to the 3' end of the single strand nicks.

7. Method according to any of the claims 1 to 6 **characterized in that** the oligonucleotide sequences coupled to the nicks are primer sequences.

8. Method according to any of the claims 1 to 7 **characterized in that** the polynucleotides are derived from synthetic or genomic DNA or RNA or a plurality of DNA or RNA molecules comprising 50 to 2000 nucleotides.

9. Method according to any of the claims 1 to 8 **characterized in that** before step a, the polynucleotides are provided at the 3' and 5' ends with primer sequences for amplification.

10. Method according to claim 8 **characterized in that** the primer sequences are same or different than the oligonucleotide sequences.

11. Method according to any of the claims 1 to 10 **characterized in that** after step c, the the target library is amplified.

12. Method according to any of the claims 1 to 11 **characterized in that** multiplying the polynucleotides in step a) is conducted by polymerase chain reaction.

13. Method according to any of the claims 1 to 12 **characterized in that** nicks are generated by a providing one or more enzymes selected from the group consisting of DNA glycosylases, endonucleases, engineered recombinant proteins and thermolabile USER II enzyme.

14. Method according to any of the claims 1 to 13 **characterized in that** the nucleic acid library is sequenced.
